# EUROPEAN PATENT APPLICATION

(11) **EP 3 318 242 A1**
(43) Date of publication of application: **09.05.2018**
(21) Application number: 16197792.1
(22) Date of filing: 08.11.2016
(51) Int. Cl.: A61H 3/00, A47C 9/02, A61F 5/01

(54) **WEARABLE SITTING POSTURE ASSISTING DEVICE AND METHOD FOR PROVIDING SAFETY FOR A PERSON**

(71) Applicant: noonee AG, 8630 Rüti ZH (CH)
(72) Inventor: GUNURA, Keith, 8003 Zürich (CH); VAFI, Daniel, 8006 Zürich (CH); JERGEN, Robin, 8134 Adliswil (CH); HUTTER, Simon, 9434 Au (CH); HUWYLER, Willy, 6330 Cham (CH)
(74) Representative: Daub, Thomas

(57) **Abstract**

For the purpose of improving safety, a wearable sitting posture assisting device is proposed, comprising at least one safety module (10a; 10; 10c), the safety module (10a; 10b; 10c) comprising at least one sensing unit (12a, 12b; 12c; 12d) configured for detecting at least one emergency condition, and the safety module (10a; 10b; 10c) comprising at least one safety unit (14a; 14b; 14c) configured for triggering at least one countermeasure for the emergency condition.

## Description

### State of the Art

The invention relates to a wearable sitting posture assisting device.

A posture assisting device is known from the document WO 2015/028373 A1.

The objective of the invention is, in particular, to provide a generic wearable sitting posture assisting device with improved characteristics regarding safety. The objective is achieved according to the invention by the features of patent claims 1 and 15, while advantageous embodiments and further developments of the invention may be gathered from the dependent claims.

### Advantages of the Invention

A wearable sitting posture assisting device is proposed, comprising at least one safety module, the safety module comprising at least one sensing unit configured for detecting at least one emergency condition, and the safety module comprising at least one safety unit configured for, in particular automatically or semiautomatically, triggering at least one countermeasure for the emergency condition.

By means of the invention user safety can be improved. A wearable sitting posture assisting device ensuring high safety can be provided. Advantageously, a user of a wearable sitting posture assisting device can be prevented from falling and/or getting hurt. Furthermore, a retrofitting capability can be achieved. In addition, a safety functionality can be added to existing devices in a cheap and/or easy and/or reliable and/or sustainable manner. Advantageously, work-related accidents can be avoided and/or prevented from occurring.

A "wearable sitting posture assisting device" is herein to be understood as a device which is configured for receiving a weight force of a person in a sitting posture or in a partly sitting posture and to transmit the weight force to a ground. In particular, an angle between a thigh and a shank of the person in the sitting posture is no greater than 130°, preferably no greater than 120° and advantageously no greater than 110° and/or no smaller than 60°, preferably no smaller than 70° and advantageously no smaller than 80°. In particular, the angle between the thigh and the shank of the person is approximately 90° in the sitting posture. In particular, an angle between a thigh and a shank of the person in the partly sitting posture is no greater than 170°, preferably no greater than 160° and advantageously no greater than 150° and/or no smaller than 100°, preferably no smaller than 110° and advantageously no smaller than 120°. In particular, the angle between the thigh and the shank of the person is approximately 130° in the partly sitting posture. It is conceivable that the partly sitting posture is a posture in which the person is leaning forward while partly bending his knees. In particular, a person wearing the wearable sitting posture assisting device is enabled to sit and/or to partly sit and/or to take a seat on the wearable sitting posture assisting device, wherein at least a part of the weight force is counteracted by the wearable sitting posture assisting device and/or wherein the person is counteracting only a fraction of the weight force using his muscles. In particular, the wearable sitting posture assisting device is configured for being worn by the person while the person is standing and/or while the person is walking. Advantageously, the wearable sitting posture assisting device is configured for supporting different sitting postures and/or partly sitting postures, which are in particular characterized by different sitting angles.

The wearable sitting posture assisting device in particular defines a sitting direction. Preferably, the person faces and/or looks in the sitting direction when sitting or partly sitting on the wearable sitting posture assisting device and facing forward. In particular, the sitting direction is oriented parallel to a floor on which the person is standing and/or sitting and/or walking when wearing the wearable sitting posture assisting device. In particular, the wearable sitting posture assisting device defines a walking direction. Preferably, the person faces in the walking direction when walking and/or standing with the wearable sitting posture assisting device and facing forward. In particular, the walking direction is oriented parallel to a floor on which the person is standing and/or sitting and/or walking when wearing the wearable sitting posture assisting device. In particular, the wearable sitting posture assisting device is only designed to receive and transmit the weight force. Preferably, the wearable sitting posture assisting device is not designed to generate a controllable force, which is provided to assist a person while walking, standing or lifting some loads. In this context, "configured" is in particular to mean specifically programmed, designed and/or equipped. By an object being configured for a certain function is in particular to be understood that the object implements and/or fulfills said certain function in at least one application state and/or operating state.

Preferably, the wearable sitting posture assisting device comprises at least one leg unit. In particular, the wearable sitting posture assisting device comprises at least one additional leg unit. In particular, the leg unit comprises at least one, preferably one, upper leg and/or at least one, preferably one, lower leg and/or at least one, preferably one, foot unit and/or at least one, preferably one, ground contact unit. Advantageously, the upper leg is connected to the lower leg, in particular via at least one, in particular one, knee joint. Preferably, the foot unit is connected to the lower leg. Advantageously, the ground contact unit is connected to the lower leg and/or to the foot unit. It is conceivable that the ground contact unit is at least partly implemented integrally with the lower leg and/or at least partly implemented integrally with the foot unit. It is also conceivable that the foot unit is at least partly implemented integrally with the lower leg. Preferably, the wearable sitting posture assisting device comprises at least one upper body wearing unit. In particular, the leg unit is connected to the upper body wearing unit, preferably via at least one connection strap. In this context, the term "a first object and a second object being at least partly implemented integrally" is in particular to mean that at least one component of the first object and at least one component of the second object are implemented integrally with each other. "Implemented integrally" is in particular to mean, in this context, connected at least by substance-to-substance bond, e.g., by a welding process, an adhesive bonding, an injection-molding process and/or by another process that is deemed expedient by a person having ordinary skill in the art. Advantageously, "implemented integrally" could in particular mean made of one piece. "Made of one piece" is, in particular, to mean, in this context, manufactured from one single piece, e.g., by production from one single cast and/or by manufacturing in a one-component or multi-component injection-molding process, and advantageously from a single blank.

Preferably, the wearable sitting posture assisting device comprises two leg units. Advantageously, the leg unit and the additional leg unit are implemented identically. It is also conceivable that the leg unit and the additional leg unit are implemented mirror-symmetrically with respect to each other. It is conceivable that the leg unit is configured for being worn on a left leg and the additional leg unit is configured for being worn at a right leg, or vice versa. Advantageously, the leg unit is configured for being worn either on a left leg or on a right leg. Further advantageously, the additional leg unit is configured for being worn on a left leg or on a right leg. Preferably, the additional leg unit is connected to the upper body wearing unit, preferably via at least one connection strap. In particular, the person wearing the wearable sitting posture assisting device is wearing the leg unit, in particular solely, on a first leg, for instance a left leg or a right leg. In particular, the person wearing the wearable sitting posture assisting device is wearing the additional leg unit, in particular solely, on a second leg, for instance a right leg or a left leg. Advantageously, the leg unit is arranged on a rear side of the leg on which the leg unit is worn. Further advantageously, the additional leg unit is arranged on a read side of the leg on which the additional leg unit is worn. In particular, the leg units of the wearable sitting posture assisting device are arranged on a rear side of the legs of the person when the person is sitting and/or partly sitting on the wearable sitting posture assisting device and/or standing and/or walking with the wearable sitting posture assisting device. Preferably, the person wearing the wearable sitting posture assisting device is wearing the upper body wearing unit on his upper body. Advantageously, the upper body wearing unit is implemented as a belt and/or as braces and/or as suspenders. The wearable sitting posture assisting device enables the person wearing it to walk around, stand, take a seat on the wearable sitting posture assisting device if required or wanted and stand up after sitting or partly sitting on the wearable sitting posture assisting device.

Preferably, the upper leg comprises at least one thigh connection unit for connecting to a thigh of the person. Preferably, the thigh connection unit features at least one thigh strap. In particular, the upper leg comprises a seat unit configured for providing at least one sitting surface for the person, in particular in case the person is sitting or partly sitting on the wearable sitting posture assisting device, preferably for the thigh and/or at least a lower portion of a buttock of the person, wherein "buttock" is preferably to mean one cheek of the buttocks. Preferably, the seat unit comprises at least one sitting element which features the sitting surface. Advantageously, the seat unit is in contact with the thigh of the person in case the person is sitting or partly sitting on the wearable sitting posture assisting device. Preferably, the seat unit is arranged on a rear side of the thigh of the person in case the person is standing or walking with the wearable sitting posture assisting device.

Advantageously, the upper leg comprises at least one upper leg support. Preferably, the seat unit is connected to the upper leg support. Advantageously, the thigh connection unit and/or the thigh strap is connected to the upper leg support. In particular, the upper leg support is implemented as a frame element. Preferably, the upper leg support is implemented as an elongated element. Advantageously, the upper leg features at least one upper leg longitudinal axis which is oriented at least substantially parallel to a longitudinal axis of the thigh of the person. Preferably, a main extension direction of the upper leg support is oriented at least substantially parallel, or parallel, to the upper leg longitudinal axis. In particular, the upper leg support is at least partly, preferably at least to a large extent, advantageously completely made of plastic. It is also conceivable that the upper leg support is at least partly, preferably at least to a large extent, advantageously completely made of metal, in particular made of a light metal or a light alloy, for instance aluminum and/or titanium and/or beryllium and/or scandium or other suitable metals. It is further conceivable that the upper leg support is at least partly, preferably at least to a large extent, advantageously completely made of a composite material, in particular a fiber reinforced composite material and/or a fiber reinforced plastic and/or a carbon fiber reinforced material and/or a carbon fiber reinforced polymer and/or a fiber reinforced thermoplastic. The term "at least to a large extent" is in particular to mean to an extent of at least 55 %, preferably to an extent of at least 65 %, further preferably to an extent of at least 75 %, advantageously to an extent of at least 85 % and further advantageously to an extent of at least 95 %. In this context "at least substantially parallel" is in particular to be understood as an orientation of a direction with respect to a reference direction, in particular in a plane, wherein the direction has a deviation from the reference direction in particular of less than 15º, advantageously of less than 10º and particularly advantageously of less than 2º. A "main extension direction" of an object is, in particular, to be understood, in this context, as a direction extending in parallel to a largest side of an imaginary rectangular cuboid which only just entirely encloses the object.

Preferably, the lower leg is arranged at a rear side of the shank of the person. In particular, the lower leg comprises at least one lower leg support. Advantageously, the lower leg support is implemented as a frame element. Preferably, the lower leg support is implemented as an elongated element. In particular, the lower leg features at least one lower leg longitudinal axis which is oriented at least substantially parallel to a longitudinal axis of the shank of the person. Preferably, a main extension direction of the lower leg support is oriented at least substantially parallel, or parallel, to the lower leg longitudinal axis. Advantageously, the lower leg longitudinal axis is oriented at least substantially parallel, or parallel, to the upper leg longitudinal axis.

Preferably, the upper leg and the lower leg together define a sitting angle. Advantageously, the sitting angle is an angle included between the upper leg longitudinal axis and the lower leg longitudinal axis, in particular on a rear side of the upper leg and the lower leg. In particular, in the sitting posture the sitting angle is no greater than 130°, preferably no greater than 120° and advantageously no greater than 110° and/or no smaller than 60°, preferably no smaller than 70° and advantageously no smaller than 80°. In particular, the sitting angle is approximately 90° in the sitting posture. In particular, in the partly sitting posture the sitting angle is no greater than 170°, preferably no greater than 160° and advantageously no greater than 150° and/or no smaller than 100°, preferably no smaller than 110° and advantageously no smaller than 120°. In particular, the sitting angle is approximately 130° in the partly sitting posture. Preferably, the sitting angle equals the angle between the thigh and the shank of the person. In particular, the sitting angle is approximately 180° in a standing posture.

In particular, the knee joint is pivotably connecting the lower leg to the upper leg, preferably pivotably about a knee joint axis. Advantageously, the knee joint axis is oriented at least substantially perpendicularly, or perpendicularly, to the upper leg longitudinal axis and/or to the lower leg longitudinal axis. Preferably, the knee joint axis is oriented at least substantially perpendicularly, or perpendicularly, to the sitting direction. Advantageously, the upper leg support and the lower leg support together implement at least a portion of the knee joint, or the knee joint. Preferably, a value of the sitting angle corresponds to a value of a knee joint position of the knee joint. In this context "at least substantially perpendicular" is in particular to be understood as an orientation of a direction with respect to a reference direction, in particular in a plane, wherein the direction and the reference direction include an angle, which angle deviates from an angle of 90° by no more than 15º, advantageously by no more than 10º and particularly advantageously by no more than 2º.

Preferably, the wearable sitting posture assisting device comprises at least one locking unit, which is configured for locking the upper leg with respect to the lower leg and/or the knee joint in a certain sitting angle and/or is configured for defining a smallest sitting angle. Advantageously, the locking unit is configured for locking the knee joint in different sitting angles, and/or for defining different smallest sitting angles, which sitting angles can be preferably chosen by the person. It is conceivable that the locking unit is configured for allowing to increase the sitting angle in a locked state. In particular, the person is enabled to stand up when the locking unit is in the locked state. Preferably, the locking unit is configured for enabling the person to sit down again at the defined smallest sitting angle after standing up with the locking unit still defining the same smallest sitting angle. Advantageously, the locking unit comprises at least one blocking element which is configured for blocking and/or unblocking the knee joint and/or for locking the sitting angle and/or for defining a smallest sitting angle. Preferably, the blocking element is implemented as a spring, in particular as a gas spring. Advantageously, the blocking element is connected to the upper leg, in particular to the upper leg support, and to the lower leg, in particular to the lower leg support. Preferably, the blocking element is configured for damping a movement of the upper leg with respect to the lower leg during sitting down and/or during standing up. Advantageously, the locking unit features at least one actuation element, which is configured for actuating the blocking element. Preferably, the actuation element is configured for enabling the person to block or unblock the blocking element. Advantageously, the actuation element is a mechanical actuation element. It is also conceivable that the actuation element is an electronic actuation element. It is further conceivable that the locking unit comprises at least one control unit which is configured for detecting a sitting down condition when the person is sitting down and/or detecting a standing up condition when the person is standing up and/or trigger the actuation element according to a requirement for a blocking of the blocking element.

In particular, the foot unit is configured for connecting to a shoe and/or to a foot of the person and/or the foot unit is connected to a shoe and/or to a foot of the person. Preferably, the foot unit comprises at least one shoe connector for connecting to the foot and/or to the shoe of the person. Advantageously, the foot connector features at least one shoe strap. In particular, the foot connector features at least one upper strap, which advantageously runs across an instep of the foot or of the shoe the foot unit is connected to. Preferably, the foot connector features at least one lower strap, which advantageously runs across a sole of the foot or of the shoe the foot unit connected to. Preferably, the foot connector is configured for being worn on a shoe and/or on a foot. Advantageously, the foot unit comprises at least one foot unit support. Preferably, the shoe connector is connected to the foot unit support. Advantageously, the foot unit support comprises at least one bracket and/or the foot unit support element is implemented as a bracket. Preferably, the shoe strap, in particular the upper strap and/or the lower strap, is connected to the bracket. It is conceivable that the foot unit support element is at least partly implemented integrally, or implemented integrally, with the lower leg, in particular with the lower leg support.

In particular, the ground contact unit comprises at least one ground contact element. Preferably, the ground contact element features at least one ground contact surface, which is advantageously configured for contacting a ground when the person is sitting or partly sitting on the wearable sitting posture assisting device. Advantageously, the ground contact surface is bent and/or curved, in particular convexly bent and/or convexly curved. Preferably, at least a portion of the ground contact element or the entire ground contact element is ellipsoidally and/or spheroidally and/or spherically shaped. In particular, the ground contact element is at least partly, preferably at least to a large extent, advantageously completely made of rubber. Preferably, a weight force of the person is transmitted from the seat unit to the upper leg support and/or from the upper leg support to the knee joint and/or from the knee joint to the lower leg support and/or from the lower leg support to the ground contact element and/or from the ground contact element to the ground. In particular, the weight of the person is additionally transmitted to the ground via the foot or shoe of the person. Preferably, the ground contact element is arranged on a rear side of the shoe of the person. When the person is sitting or partly sitting on the wearable sitting posture assisting device, the foot and/or the shoe of the person is in contact with the ground in addition to the ground contact element. Preferably, the ground contact element is arranged contactlessly with respect to the ground when the person is walking or standing while wearing the sitting posture assisting device.

In particular, the emergency condition is a falling condition of the person. In particular, the person is tripping and/or falling and/or tipping and/or is about to trip and/or about to fall and/or about to tip in the emergency condition. The person is in particular about to fall backwards in the emergency condition, in particular while or after sitting and/or while or after partly sitting on the wearable sitting posture assisting device. Advantageously, the countermeasure includes a generation of at least one counterforce counteracting a falling and/or a tripping and/or a tipping of the person.

Preferably, the safety module comprises at least one housing which covers a portion of the safety module, in particular at least a portion of the safety unit. Advantageously, the housing is part of the safety unit. Preferably, the safety module comprises at least one, advantageously electrical switch for turning the sensing unit and/or the safety module on or off.

Advantageously, the safety unit is separate from the sensing unit. In particular, the safety unit is arranged on or in a different element or unit of the wearable sitting posture assisting device as the sensing unit. Preferably, the sensing unit is arranged on or in the lower leg and/or on or in the ground contact unit and/or on or in the foot unit. Preferably, the safety unit and the sensing unit are communicatingly connected, for instance via at least one wireless connection and/or via at least one electrical connection and/or via at least one mechanical connection. In particular, the sensing unit is communicating with the safety unit in the emergency case, preferably upon detection of the emergency case. The sensing unit is triggering and/or actuating the safety unit in the emergency case, preferably upon detection of the emergency case.

In particular, the safety module is connected to the leg unit. Preferably, the wearable sitting posture assisting device comprises at least one additional safety module. Advantageously, the additional safety module is connected to the additional leg unit. Preferably, the additional safety module is implemented identically to the safety module. It is also conceivable that the wearable sitting posture assisting device comprises only one safety module. In particular, in this case it is conceivable that the safety module is connected to only one of the leg units of the wearable sitting posture assisting device.

For the purpose of preventing a person from falling it is proposed that the safety unit comprises at least one deployable safety element configured for at least partly carrying a weight of the person in the emergency condition. Preferably, the safety element is arranged at least partly, advantageously entirely, inside the housing in a normal wearing condition and/or in a taken off condition. In particular, the normal wearing condition encompasses the person wearing the wearable sitting posture assisting device in absence of an emergency case, for instance while walking and/or while standing and/or while sitting and/or while partly sitting and/or while sitting down and/or while standing up. Preferably, the safety element or at least a portion of the safety element is configured for being ejected from the housing in the emergency condition. Advantageously, the safety element or at least a portion of the safety element moves towards the ground during deployment. Preferably, the safety element carries the weight of the person together with the leg unit and the additional leg unit. In particular, the safety element carries at least 10 %, preferably at least 20 %, further preferably at least 30 %, advantageously at least 40 % and further advantageously at least 50 % of the weight of the person in the emergency condition. In particular, the safety element is configured for carrying up to 20 kg, preferably up to 50 kg, further preferably up to 100 kg and advantageously up to 200 kg of weight in the emergency condition. In particular, the safety unit is configured for deploying the safety element within no more than 2000 ms, preferably within no more than 1000 ms, further preferably within no more than 500 ms, advantageously within no more than 200 ms and further advantageously within no more than 100 ms, or even faster.

For the purpose of achieving fast ejection of a safety element in an emergency case it is proposed that the safety element is at least partly self-extracting. Preferably, the safety element extracts towards the ground in the emergency condition.

High stability and/or a high degree of reliability can be achieved if the safety element comprises at least one extendable leg. Preferably, the extendable leg is implemented as a telescopic leg. In particular, the extendable leg is configured for snapping in an extended position, preferably in a manner that the extendable leg is prevented from being compressed by the weight force of the person. In particular, the extendable leg comprises at least two, preferably at least three, further preferably at least four, or more, for instance five or six or seven or eight or nine or ten or more, stage elements, wherein preferably a first stage element is slidably mounted to a second stage element, the second stage element is preferably slidably mounted to the third stage element, etc. Preferably, in an extended state the stage elements are locked with respect to each other, advantageously in a manner that the extendable leg can withstand at least a part of the weight force of the person in the emergency condition. Advantageously, the extendable leg protrudes from the upper leg and/or from the lower leg at least substantially perpendicularly or perpendicularly or obliquely. In particular, a longitudinal axis of the extendable leg and the upper leg longitudinal axis include an angle of at least 30°, preferably of at least 40°, further preferably of at least 50°, advantageously of at least 60°, further advantageously of at least 70°, in particular in case the safety unit is connected to the upper leg. In particular, a longitudinal axis of the extendable leg and the lower leg longitudinal axis include an angle of at least 20°, preferably of at least 30°, further preferably of at least 40°, advantageously of at least 50°, further advantageously of at least 60°, in particular in case the safety unit is connected to the lower leg. In particular, the wearable sitting posture assisting device is a tripod or a quadruped in the emergency case, in particular after the safety element or the safety elements have been deployed.

For the purpose of achieving a high degree of reliability of deployment of a safety element, it is proposed that the safety element comprises at least one inflatable safety balloon. Advantageously, the safety balloon is self-inflating. In particular, the safety element comprises at least one cartridge for inflating the safety balloon. Advantageously, the cartridge is implemented as a pressurized gas cartridge, in particular as a pressurized air cartridge. Preferably, the sensing unit is configured for triggering inflation of the safety balloon upon detection of the emergency condition. Advantageously, the safety balloon is configured for propelling a portion of the safety element. It is also conceivable that the safety balloon is configured for at least partly carrying the weight of the person in the emergency condition. In particular, the safety balloon may be configured for contacting the ground in the emergency condition. In this case, the safety balloon advantageously inflates in the emergency case and functions in the manner of an airbag. Furthermore, in this case, the air balloon in particular features an inflated volume of at least 10 I, preferably of at least 20 I, further preferably of at least 30 I, advantageously of at least 40 I and further advantageously of at least 50 I. In this case, in particular, the air balloon is configured for stopping and/or slowing down a falling, in particular a falling backwards, of the person in the emergency case.

For the purpose of rapid extension of a safety leg it is proposed that the safety balloon is at least partly, preferably entirely, positioned inside the extendable leg and is configured for generating at least one extension force for extending the extendable leg. Preferably, the safety balloon inflates into the extendable leg in the emergency case. In particular, the safety balloon is configured for extending the extendable leg owing to the extension force. In particular, the cartridge is at least partly, preferably entirely, arranged inside the extendable leg. Preferably, the cartridge is arranged at a top end of the extendable leg. Advantageously, the safety balloon inflates into the extendable leg towards a lower end of the extendable leg, which is in particular configured for contacting the ground. Preferably, the lower end of the extendable leg comprises at least one foot element, which is advantageously made of rubber. It is also conceivable that the cartridge is configured for being pushed towards the lower end of the extendable leg in the emergency case, in particular in order to extend the extendable leg.

It is further proposed that the safety element is swivel-mounted, in particular to the leg unit and/or to the upper leg support and/or to the housing and/or to a support element of the safety unit and/or with respect to the leg unit, about at least one swivel axis. Preferably, the swivel axis is oriented at least substantially parallel, or parallel, to the knee joint axis. Preferably, the safety element features at least one safety element longitudinal axis. Advantageously, the safety element longitudinal axis is defined by a main extension direction of the extendable leg. In particular, the safety element longitudinal axis is oriented at least substantially parallel, or parallel, to the upper leg longitudinal axis in the normal wearing condition, in particular in case the safety unit is mounted to the upper leg. It is also conceivable that the safety element longitudinal axis is oriented at least substantially parallel, or parallel, to the lower leg longitudinal axis, in particular in case the safety unit is mounted to the lower leg. In particular, the safety element is in at least one basic position in the wearing condition. As a result, a safety unit can be connected to a leg unit in a space-efficient manner.

In a preferred embodiment of the invention it is proposed that the safety unit comprises at least one activation element configured for at least partly generating a swivel force on the safety element in the emergency condition. Preferably, the sensing unit triggers the activation element upon detection of the emergency condition. Advantageously, the activation element is configured for swiveling the safety element into an emergency position, preferably before the safety balloon is inflated and/or before the extendable leg is extended. In particular, the emergency position is different from the basic position. Preferably, an angle included by the safety element longitudinal axis and the upper leg longitudinal axis and/or an angle included by the safety element longitudinal axis and the lower leg longitudinal axis equals an angle included by the longitudinal axis of the extended leg and the upper leg longitudinal axis and/or an angle included by the longitudinal axis of the extendable leg and the lower leg longitudinal axis, respectively. In particular, deployment of the safety element encompasses swiveling the safety element into the emergency position and inflating the safety balloon. It is conceivable that the safety unit comprises at least one locking element for the activation element. In particular, the locking element is configured for preventing the activation element from generating the swivel force. Preferably, the locking element locks a position of the safety element with respect to the leg unit. For instance, the locking element may be implemented as a magnetic lock and/or as a mechanic lock and/or as a locking strap. Advantageously, the sensing unit is configured for triggering an unlocking of the locking element in order to trigger the generation of the swivel force by the activation element. As a result a fast and reliable deployment mechanism for a safety element can be achieved.

For the purpose of a high degree of user-friendliness and/or easy handling it is proposed that the activation element is pre-loadable by at least one movement applied to the safety element. In particular, the activation element is pre-loadable by moving the safety element from the emergency position into the basic position. Preferably, the safety element snaps into place in the basic position. Advantageously, the activation element is configured for storing at least a part of a loading force exerted onto the safety element during pre-loading, in particular in order to generate the swivel force in the emergency condition.

A high degree of reliability and/or simplicity of design can be achieved if the activation element is implemented as a torsion spring. Preferably, the activation element is connected to the housing.

It is further proposed that the sensing unit features at least one tilt detector and/or at least one acceleration detector for detecting the emergency condition.

Preferably, the sensing unit features at least one, in particular electronic, control unit which is configured for processing at least one detector signal of the tilt detector and/or of the acceleration detector. In particular, the control unit is configured for determining the emergency condition based on the detector signal. Advantageously, the control unit is configured for distinguishing between tilt values and/or acceleration values that are characteristic for the emergency condition and tilt values and/or acceleration values that are characteristic for the normal wearing condition. Preferably, the control unit is configured for triggering the safety unit in the emergency condition. As a result, a condition, in which a person wearing a sitting posture assisting device is falling/tipping/tripping or is about to fall/tip/trip, can be detected fast and/or reliably.

For the purpose of avoiding errors when detecting an emergency condition it is proposed that the sensing unit features at least one sitting detector, which is configured for detecting at least one sitting condition, in which sitting condition the person is sitting on the wearable sitting posture assisting device. In particular, the sensing unit is configured for triggering the safety unit only in case the person is sitting on the wearable sitting posture assisting device or was sitting on the wearable sitting posture assisting device immediately previously, in particular no more than 1 s ago, preferably no more than 0,5 s ago and advantageously no more than 0,2 s ago.

Preferably, the safety unit is mounted to the leg unit. Advantageously, the safety unit is mounted to the upper leg of the leg unit. It is also conceivable that the safety unit is mounted to the lower leg. Preferably, a safety unit of the additional safety module is mounted to the additional leg analogously to the safety unit of the safety module being mounted to the leg unit. Advantageously, the tilt detector and/or the acceleration detector and/or the sitting detector is mounted to the lower leg and/or to the foot unit and/or to the ground contact unit. In particular, the sitting detector may be integrated in the ground contact element. It is conceivable that the tilt detector is implemented as a mechanical tilt detector. Furthermore, it is conceivable that the sensing unit is implemented fully mechanically and/or configured for mechanically triggering the safety unit.

A high degree of user safety and/or retrofitting capability can be achieved with a safety module for a wearable sitting posture assisting device, in particular for a leg unit of a wearable sitting posture assisting device, according to the invention.

Furthermore, the invention is concerned with a method for providing safety for a person, in particular in a fabrication line, a factory building, a service building or an office building, wherein the person is wearing a wearable sitting posture assisting device.

It is proposed that in at least one emergency condition at least one countermeasure for the emergency condition is, in particular automatically or semiautomatically, triggered.

By means of the invention user safety can be improved. A wearable sitting posture assisting device ensuring high safety can be provided. Advantageously, a user of a wearable sitting posture assisting device can be prevented from falling and/or from getting hurt. Furthermore, a retrofitting capability can be achieved. In addition, a safety functionality can be added to existing devices in a cheap and/or easy and/or reliable and/or sustainable manner. Advantageously, work-related accidents can be avoided and/or prevented from occurring.

Herein, the wearable sitting posture assisting device and the method according to the invention are not to be limited to the application and implementation described above. In particular, for the purpose of fulfilling a functionality herein described, the wearable sitting posture assisting device and the method according to the invention may comprise a number of respective elements, structural components, units and/or steps that differ from the number mentioned herein. Furthermore, regarding the value ranges mentioned in this disclosure, values within the limits mentioned are to be understood to be also disclosed and to be used as applicable.

### Drawings

Further advantages may become apparent from the following description of the drawing. In the drawing exemplary embodiments of the invention are shown. The drawing, the description and the claims contain a plurality of features in combination. The person having ordinary skill in the art will purposefully also consider the features separately and will find further expedient combinations.

If there is more than one specimen of a certain object, only one of these is given a reference numeral in the figures and in the description. The description of this specimen may be correspondingly transferred to the other specimens of the object.

It is shown in :
- Fig. 1: a person wearing a wearable sitting posture assisting device, in a schematic lateral view,
- Fig. 2: the person wearing the wearable sitting posture assisting device, in a schematic front view,
- Fig. 3: the person wearing the wearable sitting posture assisting device in a schematic rear view,
- Fig. 4: a leg unit of the wearable sitting posture assisting device comprising a safety module, in a normal wearing condition, in a schematic lateral view,
- Fig. 5: the leg unit in an emergency condition, in a schematic lateral view,
- Fig. 6: a portion of a safety unit of the safety module, in a schematic lateral sectional view,
- Fig. 7: a portion of the safety unit in a schematic lateral view,
- Fig. 8: a schematic flow chart of a method for providing safety for the person,
- Fig. 9: an alternative wearable sitting posture assisting device comprising a leg unit and a safety module, in a normal wearing condition, in a schematic lateral view,
- Fig. 10: the leg unit of the alternative wearable sitting posture assisting device, in an emergency condition, in a schematic lateral view,
- Fig. 11: a portion of a safety unit of the safety module of the alternative wearable sitting posture assisting device, in a schematic lateral sectional view,
- Fig. 12: a further alternative wearable sitting posture assisting device, in an emergency condition, in a schematic lateral view.
- Fig. 13: an alternative sensing unit for a wearable sitting posture assisting device in a schematic partial sectional lateral view.

### Description of the exemplary embodiments

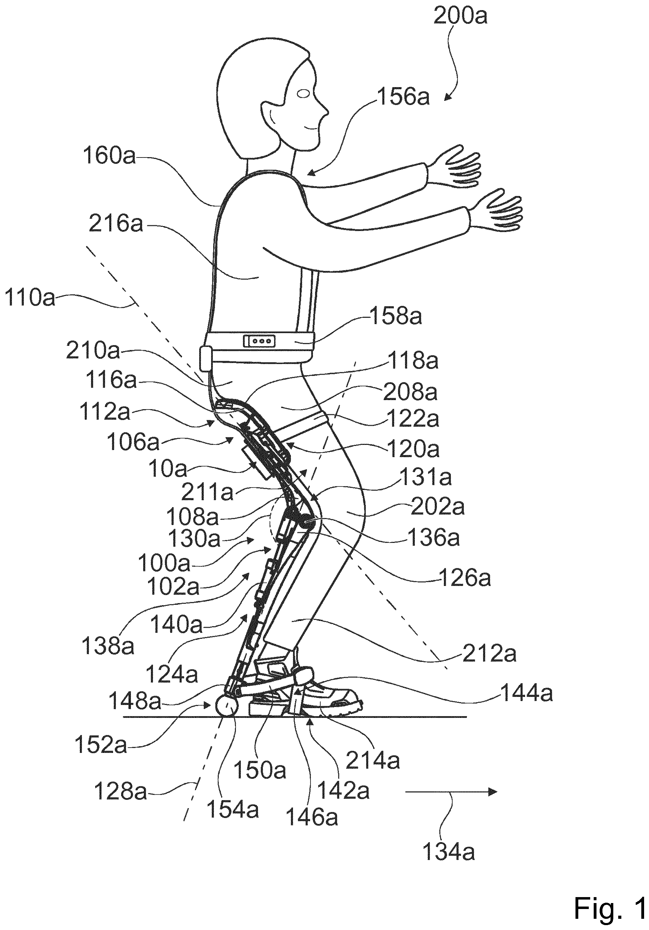

Fig. 1 shows a person 200a wearing a wearable sitting posture assisting device 100a. The wearable sitting posture assisting device 100a is configured for receiving a weight force of the person 200a in a sitting posture or in a partly sitting posture. In fig. 1 the person 200a is shown in a partly sitting posture. In the partly sitting posture a knee 202a of the person is partly bent. In a sitting posture the knee 202a is bent more strongly than in the partly sitting posture. The wearable sitting posture assisting device 100a is configured for allowing the person 200a to sit down on it in different sitting postures and in different partly sitting postures. Furthermore, the wearable sitting posture assisting device 100a is configured for allowing the person 200a to walk while wearing the wearable sitting posture assisting device 100a. In addition, the wearable sitting posture assisting device 100a is configured for allowing the person 200a to stand and/or stand up and/or sit down and/or walk while wearing the wearable sitting posture assisting device 100a.

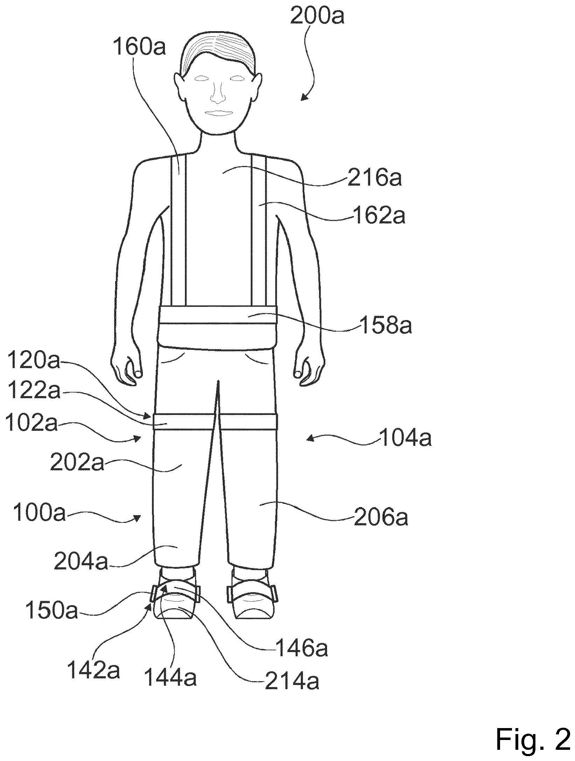

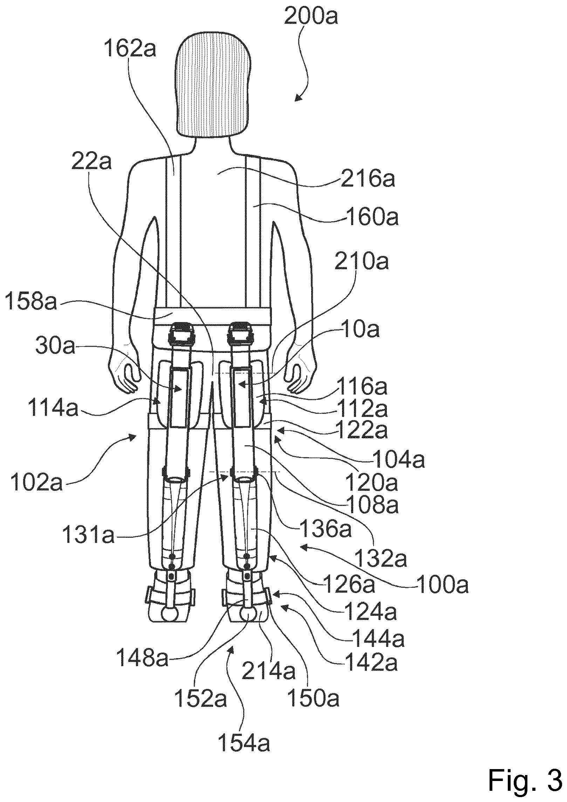

Fig. 2 shows the person 200a wearing the wearable sitting posture assisting device 100a, in a schematic front view. Fig. 3 shows the person 200a wearing the wearable sitting posture assisting device 100a, in a schematic rear view. In figs 1 to 3 the wearable sitting posture assisting device 100a is shown in a normal wearing condition. The normal wearing conditions encompasses a condition in which the person 200a is sitting or partly sitting on the wearable sitting posture assisting device 100a, a condition in which the person 200a is standing up, a condition in which the person 200a is sitting down, a condition in which the person 200a is standing, and a condition in which the person 200a is walking, in each case while wearing the wearable sitting posture assisting device 100a. In the shown case the person 200a is wearing the wearable sitting posture assisting device 100a in a factory building, in particular while working on an assembly line. In a similar fashion it is conceivable that the person 200a wears the wearable sitting posture assisting device 100a in an office building, in a factory building, in a service building, outside, at work, at home, while working, during breaks, etc. Advantageously, the person 200a wears the wearable sitting posture assisting device 100a during an activity which requires the person 200a to sit down and/or to partly sit down and/or to stand up and/or to stand and/or to walk repeatedly. The person 200a can then sit down on the wearable sitting posture assisting device 100a when required, stand up while wearing the wearable sitting posture assisting device 100a when required, and walk while wearing the wearable sitting posture assisting device 100a when required.

The wearable sitting posture assisting device 100a comprises a leg unit 102a. Furthermore, the wearable sitting posture assisting device 100a comprises an additional leg unit 104a. The additional leg unit 104a is implemented identically to the leg unit 102a. Therefore, in the following, only the leg unit 102a is described in detail. The description of the leg unit 102a is to be understood as transferable to the additional leg unit 104a. It is also conceivable that an additional leg unit is implemented mirror-symmetrically to the leg unit. In particular, it is conceivable that a leg unit and an additional leg unit are implemented as right leg unit and left leg unit, respectively, or vice versa.

In the case shown, the person 200a is wearing the leg unit 102a on a right leg 204a. The leg unit 102a is arranged on a rear side 211 a of the leg 204a of the person 200a. Furthermore, the person 200a is wearing the additional leg unit 104a on a left leg 206a. It is also conceivable that a person wears a leg unit on a left leg and an additional leg unit on a right leg. Furthermore, it is conceivable that a person only wears one leg unit. In addition, it is conceivable that a wearable sitting posture assisting device comprises only one leg unit. It is also conceivable that a leg unit is arranged on a lateral side of a leg and/or on a front side of a leg and/or between two legs of a person.

The person 200a is sitting or partly sitting on the wearable sitting posture assisting device 100a in a sitting direction 134a. The person 200a faces and/or looks in the sitting direction 134a when facing forward. The sitting direction 134a is oriented parallel to a ground on which the person 200a is sitting or walking or standing.

The leg unit 102a comprises an upper leg 106a. The upper leg 106a comprises an upper leg support 108a. The upper leg 106a has an upper leg longitudinal axis 110a. The upper leg longitudinal axis 110a is oriented perpendicularly to the sitting direction 134a. The upper leg support 108a has a main extension direction which is oriented parallel to the upper leg longitudinal axis 110a. The upper leg longitudinal axis 110a is oriented parallel to a main extension direction of a thigh 208a of the leg 204a of the person, in particular when the person 200a is sitting, and/or partly sitting and/or walking and/or standing up and/or standing while wearing the wearable sitting posture assisting device 100a.

The upper leg 106a comprises a seat unit 112a. The seat unit 112a is connected to the upper leg support 108a. In the partly sitting posture and/or in the sitting posture the person 200a is sitting on the seat unit 112a. In the case shown the person 200a is sitting on the seat unit 112a and on a seat unit 114a of the additional leg unit 104a in the partly sitting posture. The seat unit 112a comprises a sitting element 116a. The sitting element 116a contacts the thigh 208a of the person 200a. Furthermore, in the sitting posture and/or in the partly sitting posture the sitting element 116a contacts a buttock 210a of the person 200a. The seat unit 112a comprises a sitting surface 118a. The sitting element 116a comprises the sitting surface 118a. The sitting surface 118a is configured for allowing the person 200a to sit down on it with the thigh 208a and/or with the buttock 210a. A shape of the sitting surface 118a is at least partly adjusted to the thigh 208a and/or to the buttock 210a of the person 200a. The sitting surface 118a is curved. The sitting surface 118a is concavely curved and/or bent.

It is also conceivable that a wearable sitting posture assisting device comprises only one seat unit, in particular a common seat unit of two leg units. In particular, in this case it is conceivable that the seat unit is saddle-shaped and/or implemented in the manner of a saddle, in particular arranged between the legs of a person.

The upper leg 106a comprises a thigh connection unit 120a for connecting to the thigh 208a of the person 200a. The thigh connection unit 120a is connected to the upper leg support 108a. The thigh connection unit 120a is configured for connecting the upper leg 106a to the thigh 208a of the person 200a. The thigh connection unit 120a comprises a thigh strap 122a. The thigh strap 122a is fixed to the thigh 208a of the person 200a.

The wearable sitting posture assisting device 100a comprises a lower leg 124a. The lower leg 124a comprises a lower leg support 126a. The lower leg 124a has a lower leg longitudinal axis 128a. The lower leg longitudinal axis 128a is oriented perpendicularly to the sitting direction. The lower leg longitudinal axis 128a and the upper leg longitudinal axis 110a are arranged in a common plane. The lower leg support 126a has a main extension direction which is oriented parallel to the lower leg longitudinal axis 128a. The lower leg longitudinal axis 128a is oriented parallel to a main extension direction of a shank 212a of the leg 204a of the person, in particular when the person 200a is sitting, and/or partly sitting and/or walking and/or standing while wearing the wearable sitting posture assisting device 100a.

The upper leg 106a and the lower leg 124a define a sitting angle 130a. The sitting angle 130a is an angle included by the upper leg longitudinal axis 110a and the lower leg longitudinal axis 128a. The sitting angle 130a is similar or identical to an angle between the thigh 208a and the shank 212a of the person 200a. The sitting angle 130a having a value between 60° and 130°, in particular a value of approximately 90°, corresponds to different sitting postures or to at least one sitting posture. The sitting angle 130a having a value between 130° and 170° corresponds to different partly sitting postures. In case the person 200a is standing while wearing the wearable sitting posture assisting device 100a the sitting angle 130a has a value between 160° and 180°, in particular a value of approximately 180°. In case the person 200a is walking while wearing the wearable sitting posture assisting device 100a the sitting angle 130a may significantly differ from 180°, in particular in case the person 200a bends his knee 202a. In the sitting posture and/or in the partly sitting posture and/or when standing the sitting angle 130a and an analogously defined additional sitting angle of the additional leg unit 104a are advantageously identical. However, it is also conceivable that the person 200a is sitting on the wearable sitting posture assisting device 100a in a sitting posture or a partly sitting posture with the sitting angle 130a and the additional sitting angle being differing, in particular by up to 5°, by up to 10°, by up to 15°, by up to 20°, by up to 30°, by up to 40°, or by more. When the person 200a is walking while wearing the wearable sitting posture assisting device 100a the sitting angle 130a and the additional sitting angle may significantly differ, for instance in case the person bends his knees differently.

The leg unit 102a comprises a knee joint 131 a which pivotably connects the upper leg 106a to the lower leg 124a. The knee joint 131 a connects the upper leg 106a to the lower leg 124a pivotably about a knee joint axis 132a. The knee joint axis 132a is oriented perpendicularly with respect to the upper leg longitudinal axis 110a. The knee joint axis 132a is oriented perpendicularly to the lower leg longitudinal axis 128a. The knee joint axis 132a is oriented perpendicularly to the sitting direction 134a. The knee joint 131 a is partly implemented integrally with the upper leg support 108a. The knee joint 131 a is partly implemented integrally with the lower leg support 126a. The knee joint 131 a comprises at least one bearing 136a which connects the upper leg support 108a to the lower leg support 126a.

The leg unit 102a comprises a locking unit 138a which is configured for locking the knee joint 131 a. The locking unit 138a is configured for limiting the sitting angle 130a to a minimum value. The locking unit 138a is configured for allowing the person 200a to choose the minimum value of the sitting angle 130a. In case the locking unit 138a is in a locked state the person 200a can sit down on the wearable sitting posture assisting device 100a with the sitting angle 130a having the minimum value. The locking unit 138a is configured for being actuated by the person 200a. The locking unit 138a comprises a blocking element 140a. The blocking element 140a is a spring, in particular a gas spring. The blocking element 140a is configured for being locked at different lengths. The blocking element 140a is connected to the upper leg support 108a. The blocking element 140a is connected to the lower leg support 126a. The blocking element 140a is configured for damping a movement of the upper leg 106a with respect to the lower leg 124a, in particular when the person 200a is sitting down.

The leg unit 102a comprises a foot unit 142a. The foot unit 142a is configured for connecting to a shoe 214a and/or to a foot of the person 200a. The foot unit 142a comprises a shoe connector 144a for connecting to the shoe 214a and/or to the foot of the person 200a. The foot connector 144a comprises a strap 146a which is fixed to the shoe 214a of the person 200a. The foot unit 142a comprises a foot unit support 148a. The foot unit support 148a is connected to the lower leg 124a. The foot unit support 148a is connected to the lower leg support 126a. The foot unit support 148a comprises a bracket 150a. The shoe connector 144a is connected to the foot unit support 148a. The shoe connector 144a is connected to the bracket 150a. The strap 146a is connected to the bracket 150a.

The leg unit 102a comprises a ground contact unit 152a. The ground contact unit 152a is connected to the foot unit 142a. The ground contact unit 152a is connected to the lower leg support 126a. The ground contact unit 152a comprises a ground contact element 154a. When the person 200a is sitting or partly sitting on the wearable sitting posture assisting device 100a the ground contact unit 152a, in particular the ground contact element 154a, is in contact with the ground. The ground contact unit 152a, in particular the ground contact element 154a, is configured for transmitting a part of the weight force of the person 200a into the ground. The ground contact element 154a is rounded. The ground contact element 154a is spherical. The ground contact element 154a is made of rubber. However, other shapes and/or materials are conceivable for a ground contact element as mentioned above.

In case the person 200a is sitting or partly sitting on the wearable sitting posture assisting device 100a the weight force of the person 200a is at least partly, in particular directly or indirectly, transmitted from the seat unit 112a to the upper leg support 108a; from the upper leg support 108a to the knee joint 131 a; from the knee joint 131 a to the lower leg support 126a; from the lower leg support 126a to the ground contact element 154a; from the ground contact element 154a to the ground.

In particular, the weight force of the person 200a is additionally transmitted to the ground via the foot or shoe 214a of the person 200a. Preferably, the ground contact element 154a is arranged on a rear of the shoe 214a of the person 200a. When the person 200a is sitting or partly sitting on the wearable sitting posture assisting device 100a the foot and/or the shoe 214a of the person 200a is in contact with the ground in addition to the ground contact element 154a. Preferably, the ground contact element 154a is arranged contactlessly with respect to the ground when the person 200a is walking and/or standing while wearing the wearable sitting posture assisting device 100a.

The wearable sitting posture assisting device 100a comprises an upper body wearing unit 156a. The person 200a is wearing the upper body wearing unit 156a on his upper body 216a, which upper body 216a may include hips and/or a waist of the person 200a. The upper body wearing unit 156a comprises a belt 158a. Furthermore, the upper body wearing unit 156a comprises suspenders 160a, 162a. The leg unit 102a is connected to the upper body wearing unit 156a. The additional leg unit 104a is connected to the upper body wearing unit 156a. It is conceivable that an upper body wearing unit comprises no belt and only suspenders, or vice versa. It is also conceivable that a wearable sitting posture assisting device is only connected to the legs and/or the feet and/or the shoes of a person wearing it.

The wearable sitting posture assisting device 100a comprises a safety module 10a. The safety module 10a is mounted to the leg unit 102a. The wearable sitting posture assisting device 100a comprises an additional safety module 30a. The additional safety module 30a is mounted to the additional leg unit 104a. The additional safety module 30a is implemented identically to the safety module 10a.

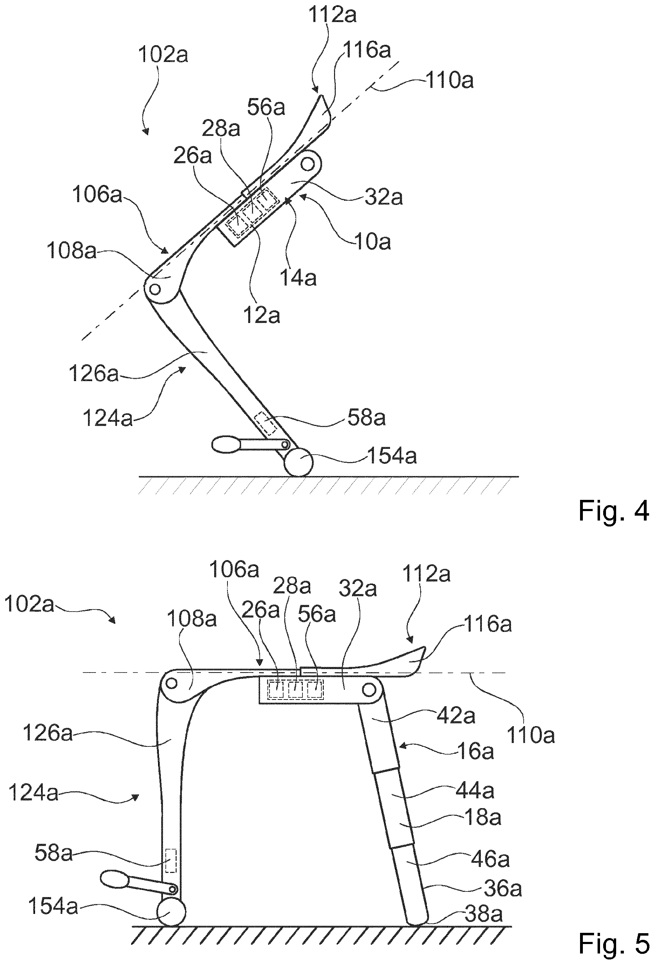

Fig. 4 shows the leg unit 102a in the normal wearing condition, in a schematic lateral view. The safety module 10a comprises a sensing unit 12a configured for detecting at least one emergency condition. The safety module 10a comprises a safety unit 14a configured for triggering at least one countermeasure for the emergency condition.

Fig. 5 shows the leg unit 102a in the emergency condition. In the case shown the emergency condition is a condition in which the person 200a is about to fall backwards or falls backwards while sitting or partly sitting on the wearable sitting posture assisting device 100a. In the case shown the person 200a was sitting with the sitting angle 130a having a value of approximately 90° prior to falling. For instance, the person 200a may have worked on an assembly line while sitting on the wearable sitting posture assisting device 100a. The person 200a may then have leaned backwards too far and therefore may have become unbalanced. The countermeasure is configured for preventing the person 200a from falling and/or tipping and/or tripping. The safety unit 14a is implemented for triggering the countermeasure within no more than 500 ms.

The safety unit 14a comprises a deployable safety element 16a configured for at least partly carrying a weight of the person 200a in the emergency condition. In the case shown the safety element 16a is configured for carrying a weight of at least 200 kg.

The safety module 10a comprises a housing 32a. The housing 32a is mounted to the leg unit 102a. The housing 32a is mounted to the upper leg support 108a. The safety unit 14a is arranged within the housing 32a in the normal wearing condition. The safety element 16a is arranged within the housing 32a in the normal wearing condition. The safety element 16a is partly deployed out of the housing 32a in the emergency condition.

The safety element 16a is at least partly self-extracting. The safety element 16a self-extracts in the emergency condition, in particular upon occurrence of the emergency condition. In the emergency condition the safety element 16a self-extracts and subsequently remains in an extracted state. The safety element 16a carries the weight of the person 200a partly in an extracted state, in the emergency condition.

The safety element 16a comprises an extendable leg 18a. The extendable leg 18a is implemented as a telescopic leg. The extendable leg 18a is extendable in an extending direction 34a in the emergency condition. The extending direction 34a is oriented parallel to a main extension direction of the extendable leg 18a.

The extendable leg 18a features a foot element 36a. The foot element 36a is in contact with the ground in the emergency condition. In the emergency condition the ground contact element 154a and the foot element 36a are in contact with the ground. The shoe 214a and/or the foot of the person 200a may be in contact with the ground or may not be in contact with the ground, in the emergency condition. The foot element 36a features a rounded tip 38a.

In the case shown the extendable leg 18a comprises three stage elements 42a, 44a, 46a. The innermost stage element 46a is the foot element 36a. Other numbers of stage elements are conceivable, for instance a number of two, four, five, six, seven, eight, nine, ten or more.

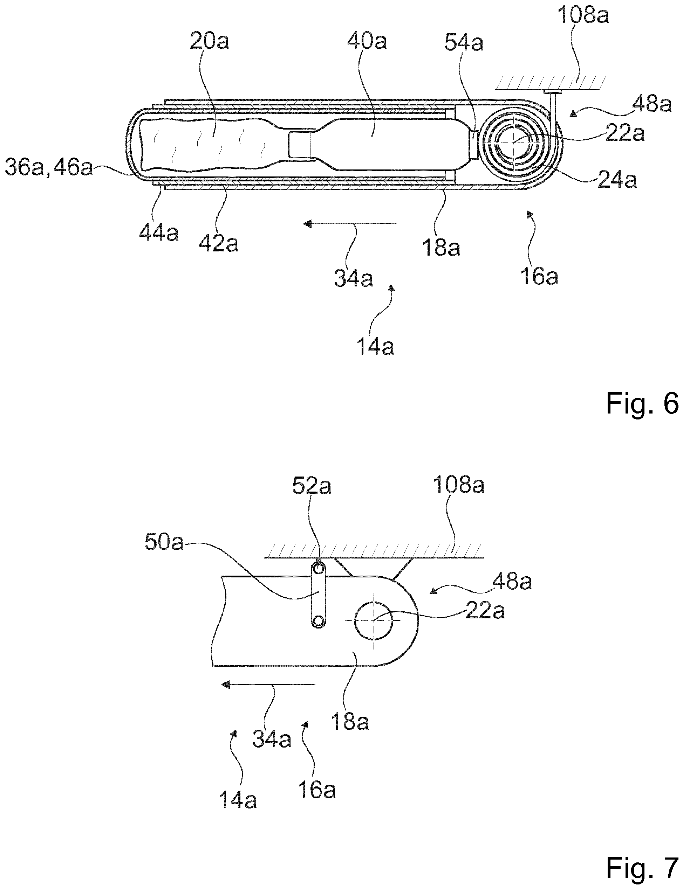

Fig. 6 shows a portion of the safety unit 14a in a schematic lateral sectional view. Fig. 7 shows a portion of the safety unit 14a in a schematic lateral view. For reasons of clarity the upper leg support 108a is only shown schematically in figs. 6 and 7.

The safety element 16a comprises an inflatable safety balloon 20a. The safety element 16a comprises a cartridge 40a for inflating the safety balloon 20a. The cartridge 40a is implemented as a pressurized air cartridge. The safety unit 14a comprises a trigger element 54a for triggering the cartridge 40a in the emergency condition. The trigger element 54a triggers an opening of the cartridge 40a and thus a release of the pressurized air into the safety balloon 20a.

The safety balloon 20a is positioned inside the extendable leg 18a. The safety balloon 20a is configured for generating at least one extension force for extending the extendable leg 18a. The safety balloon 20a is configured for generating the extension force in a manner that the extendable leg 18a is extended in the extending direction 34a. The extendable leg 18a is extended upon inflating of the safety balloon 20a by the cartridge 40a, in the emergency condition. The safety balloon 20a is configured for pushing the foot element 36a towards the ground. In the emergency condition the safety balloon 20a pushes the stage element 46a partly out of the stage element 44a and the stage element 44a partly out of the stage element 42a, thus extending the extendable leg. The stage elements 42a, 44a, 46a or at least one or at least a few of the stage elements 42a, 44a, 46a comprise at least one locking element, which is not shown, for locking a position of the respective stage element 42a, 44a, 46a with respect to at least one of the other stage elements 42a, 44a, 46a upon extending the extendable leg 18a. Thus, the extendable leg 18a may at least partly or completely snap with a length of the extendable leg 18a in its extended state being fixed due to said locking element. In the case shown the cartridge 40a is pushed towards a top end 48a of the extendable leg 18a upon inflation of the safety balloon 20a, in the emergency condition.

The safety element 16a is swivel-mounted about a swivel axis 22a (compare fig. 3). The safety element 16a is swivel-mounted with respect to the upper leg support 108a.

The safety unit 14a comprises at least one activation element 24a configured for at least partly generating a swivel force on the safety element 16a in the emergency condition. The activation element 24a is implemented as a torsion spring. The activation element 24a is directly or indirectly connected to the upper leg support 108a. In the emergency case the activation element 24a swivels the safety element 16a from a basic position into an emergency position. In the basic position the safety element 16a is oriented parallel to the upper leg longitudinal axis 110a (compare fig. 4). In the emergency condition the safety element 16a is oriented at an angle greater than 90° with respect to the upper leg longitudinal axis 110a. The safety element 16a protrudes from the upper leg 106a in the emergency position.

The activation element 24a is pre-loadable by at least one movement applied to the safety element 16a. In the case shown the activation element 24a is loadable via pushing the safety element 16a from the emergency position into the basic position. In the case shown the safety element 16a snaps into place when pushed from the emergency position into the basic position.

The safety unit 14a comprises a locking element 50a for the activation element 24a. The locking element 50a is configured for preventing the activation element 24a from generating the swivel force. The locking element 50a locks a position of the safety element 16a with respect to the leg unit 102a. The locking element 50a is implemented as a locking strap. The safety unit 14a comprises a pin element 52a The locking element 50a is connected to the pin element 52a. The pin element 52a is implemented retractably. In the emergency condition the pin element 52a retracts and releases the locking element 50a. The locking element 50a is coupled to the trigger element 54a. The trigger element 54a is triggering the cartridge 40a when the safety element 16a is in the emergency position or while the safety element 16a is swiveled into the emergency position.

The sensing unit 12a features a tilt detector 26a and an acceleration detector 28a for detecting the emergency condition. The tilt detector 26a is configured for determining an angle included by the upper leg longitudinal axis 110 and the ground. The acceleration detector 28a is configured for determining an acceleration force exerted onto the safety module 10a. It is also conceivable that a sending unit features only a tilt detector and no acceleration detector or only an acceleration detector and no tilt detector for detecting the emergency condition. Furthermore, it is conceivable that a tilt detector is arranged at a lower leg and/or is configured for determining an angle included by a lower leg longitudinal axis and a ground.

The sensing unit 12a comprises a sitting detector 58a. The sitting detector 58a is configured for detecting at least one sitting condition, in which sitting condition the person 200a is sitting on the wearable sitting posture assisting device 100a. The sitting detector 58a is configured for detecting a contact between the ground contact element 154a and the ground. The sitting detector 58a may be configured for determining a sitting force exerted onto the ground contact element 154a. In the case shown the sitting detector 58a is integrated in the lower leg support 126a. Furthermore, in the case shown the sitting detector 58a is part of the leg unit 102a. It is also conceivable that a sitting detector is implemented retrofittably to a leg unit.

Furthermore, the sensing unit comprises a control unit 56a. The control unit 56a is connected to the acceleration detector 28a. The control unit 56a is connected to the tilt detector 26a. The control unit 56a is connected to the sitting detector 58a. The control unit 56a is configured for evaluating detector signals of the tilt detector 26a and/or of the acceleration detector 28a and/or of the sitting detector 58a. The control unit 56a is configured for determining if the person 200a is falling backwards. The control unit 56a is configured for determining if the person 200a is sitting or partly sitting on the wearable sitting posture assisting device 100a. The control unit 56a is configured for triggering the safety unit 14a. In the emergency case the control unit 56a triggers the safety unit 14a and the safety element 16a is deployed.

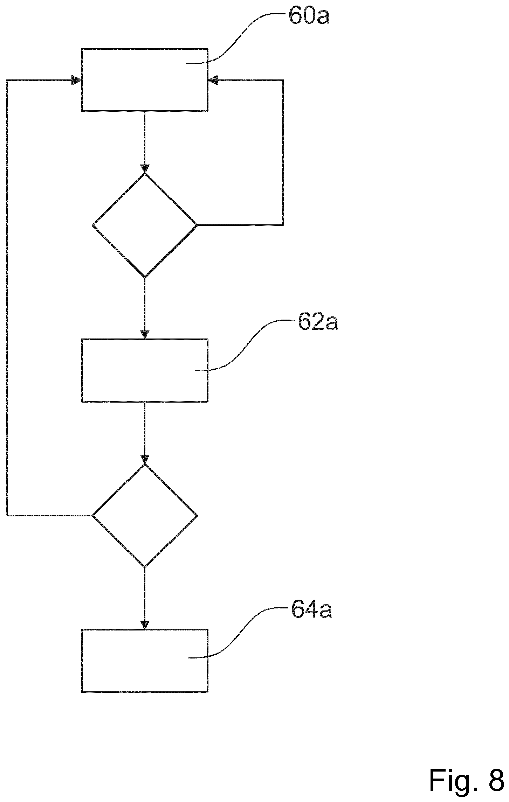

Fig. 8 shows a schematic flowchart of a method for providing safety for the person 200a wearing the wearable sitting posture assisting device 100a. The method comprises at least one method step 64a in which method step 64a, in at least one emergency condition, at least one countermeasure for the emergency condition is triggered. The safety module 10a is configured for operating according to the method.

In a first method step 60a a sitting condition of the person 200a is determined, in particular via the sitting detector 58a. In case the person 200a is not sitting or partly sitting on the wearable sitting posture assisting device 100a, the sitting condition is determined again. In case the person 200a is sitting on the wearable sitting posture assisting device 100a a falling condition is determined in a second method step 62a, in particular via the tilt detector 26a and/or via the acceleration detector 28a. In case there is no falling and/or tipping of the person 200a detected, the first method step 60a is carried out again. The method step 64a is a third method step 64a. In case a falling and/or tipping of the person 200a is detected, the third method step 64a is carried out and the countermeasure for the emergency condition is triggered. In particular, the safety element 16a is deployed in the third method step 64a.

It is also conceivable that the first method step 60a is omitted. In particular, it is conceivable that a countermeasure is also triggered in case the person is falling and/or tipping and/or tripping while standing or walking.

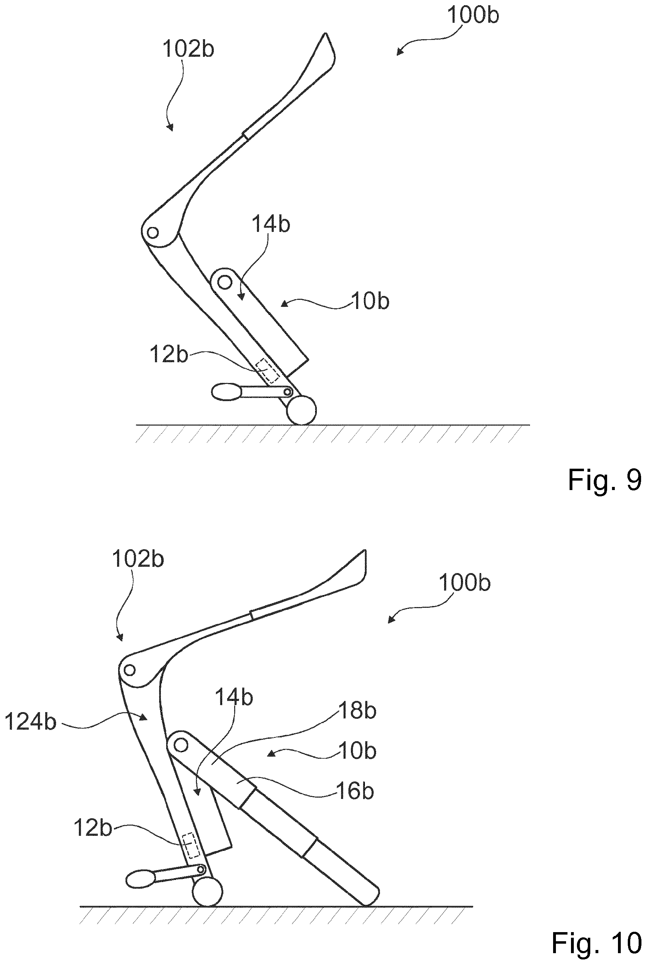

Figs. 9 to 12 show further exemplary embodiments of the invention. The following description is substantially limited to the differences between the exemplary embodiments, wherein regarding structural elements, features and functions that remain the same the description of the other exemplary embodiments, in particular the exemplary embodiment of figs. 1 to 8, may be referred to. For distinguishing the exemplary embodiments, the letter a of the reference numerals in the exemplary embodiment of figs. 1 to 8 has been substituted by the letters b and c in the reference numerals of the exemplary embodiments of figs. 9 to 12. Regarding structural elements having the same denomination, in particular regarding structural elements having the same reference numerals, principally the drawing and/or the description of the other exemplary embodiments, in particular of the exemplary embodiment of figs. 1 to 8, may be referred to.

Fig. 9 shows a leg unit 102b of an alternative wearable sitting posture assisting device 100b in a normal wearing condition, in a schematic lateral view. The leg unit 102b is implemented analogously to the leg unit 102a of the exemplary embodiment of fig. 1 to 8. The alternative wearable sitting posture assisting device 100b comprises a safety module 10b. The safety module 10b comprises a sensing unit 12b. The sensing unit 12b is configured for detecting at least one emergency condition. The safety module 10b comprises a safety unit 14b. The safety unit 14b is implemented for triggering at least one countermeasure for the emergency condition.

The safety unit 14b comprises a deployable safety element 16b configured for at least partly carrying a weight of a person wearing the wearable sitting posture assisting device 100b in the emergency condition. The safety element 16b is partly self-extracting. The safety element 16b comprises an extendable leg 18b.

Fig. 10 shows the leg unit 102b in the emergency condition, in a schematic lateral view. The safety unit 14b is mounted to a lower leg 124b of the leg unit 102b.

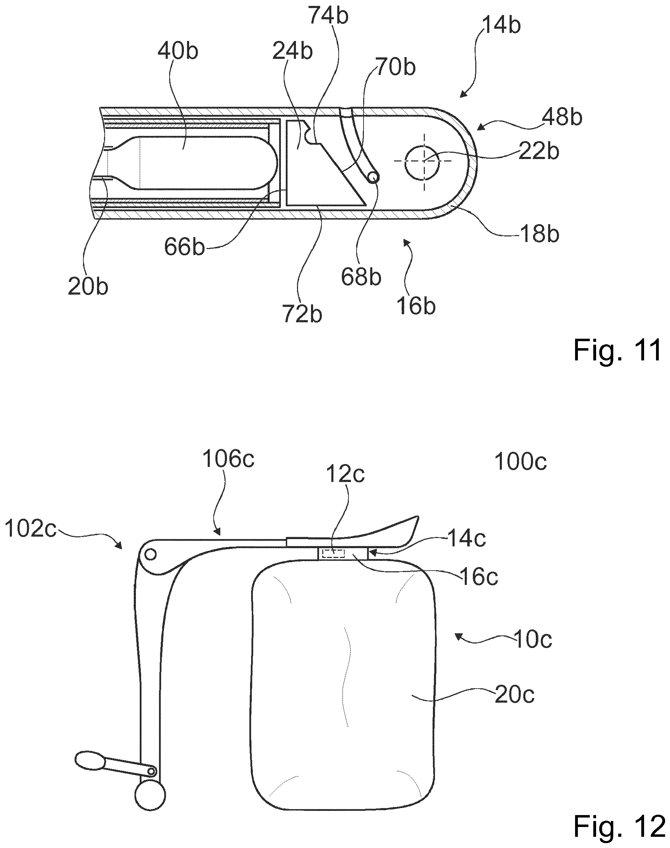

Fig. 11 shows a portion of the safety unit 14b, in a schematic lateral sectional view. The safety element 16b comprises an inflatable safety balloon 20b. The safety balloon 20b is positioned within the extendable leg 18b and is configured for generating at least one extension force for extending the extendable leg 18b. The safety element 16b comprises a cartridge 40b for inflating the safety balloon 20b. The safety element is swivel-mounted about a swivel axis 22b.

The safety unit 14b comprises an activation element 24b configured for generating a swivel force on the safety element 16b in the emergency condition. The activation element 24b is triangularly shaped. The activation element 24b is shaped in a manner of a prism, in particular a triangular prism. The activation element 24b is arranged within the extendable leg 18b. The activation element 24b is movable relative to the extendable leg 18b. The activation element 24b comprises a pushed surface 66b. In the emergency case the cartridge 40b is pushed against the pushed surface 66b when the safety balloon 20b is inflated. In the emergency case the activation element 24b is pushed towards a top end 48b of the extendable leg 18b. The safety unit 14b comprises a pin element 68b which is fixed with respect to the leg unit 102b. The safety element 16b moves with respect to the pin element 68b when swiveled about the swivel axis 22b.

The activation element 24b comprises a sliding surface 70b. The sliding surface 70b is pushed against the pin element 68b when the cartridge 40b is pushed against the pushed surface 66b. The sliding surface 70b is oriented inclined with respect to a main extension direction of the extendable leg 18b. The sliding surface 70b slides on the pin element 16b when the cartridge 40b is pushed against the pushed surface 66b.

The activation element 24b comprises a pushing surface 72b. The pushing surface 72b is oriented inclined with respect to the sliding surface 70b. The pushing surface 72b is oriented parallel to the main extension direction of the extendable leg 18b. The pushing surface 72b is pushed against the extendable leg 18b and generates the swivel force when the sliding surface 70b slides on the pin element 68b. The sliding surface 70b implements a locking position 74b for the pin element 68b. The locking position 74b defines an orientation of the extendable leg 18b with respect to the lower leg 124b in an emergency position of the extendable leg 18b.

It is conceivable that an activation element, which is implemented analogously to the activation element 24b of the exemplary embodiment of figs. 9 to 11, is used in a wearable sitting posture assisting device which is implemented analogously to the wearable sitting posture assisting device 100a of the exemplary embodiment of figs. 1 to 8, or vice versa.

Fig. 12 shows a leg unit 102c of a further alternative wearable sitting posture assisting device 100c, in a schematic lateral view. The leg unit 102c is shown in an emergency condition. The wearable sitting posture assisting device 100c comprises a safety module 10c. The safety module 10c is mounted to the leg unit 102c. The safety module 10c is mounted to a upper leg 106c of the leg unit 102c. The safety module 10c comprises a sensing unit 12c configured for detecting at least one emergency condition. The safety module 10c comprises a safety unit 14c. The safety unit 14c is configured for triggering at least one countermeasure for the emergency condition.

The safety unit 14c comprises a deployable safety element 16c configured for at least partly carrying a weight of a person 200c in the emergency condition. The safety element 16c is partly self-extracting.

The safety element 16c comprises an inflatable safety balloon 20c. In the emergency condition the safety balloon 20c is in contact with the ground. The safety balloon 20c functions in the manner of an airbag, in the emergency condition. The safety balloon 20c prevents a person wearing the wearable sitting posture assisting device 100c from falling or tipping backwards and/or decelerates the person when falling and/or tipping.

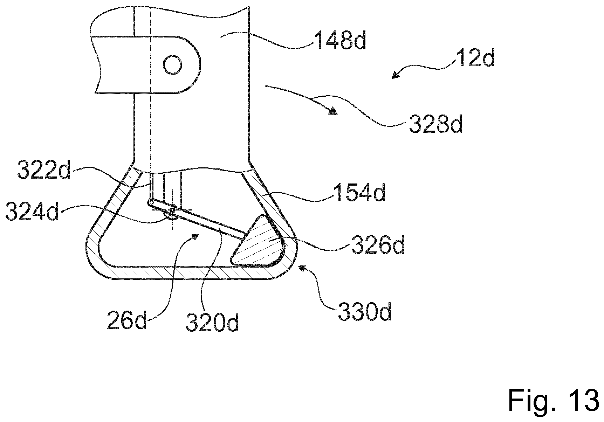

Fig. 13 shows an alternative sensing unit 12d in a schematic partial sectional lateral view. The alternative sensing unit 12d is implemented fully mechanically. The alternative sensing unit 12d is configured for mechanically triggering a safety unit of a wearable sitting posture assisting device, for instance a safety unit implemented analogously to the exemplary embodiments of figs. 1 to 12. The alternative sensing unit 12d comprises a tilt detector 26d. The tilt detector 26d is implemented mechanically. The tilt detector 26d is at least partly, in particular entirely arranged within a ground contact element 154d. The ground contact element 154d is made of a deformable material. In the case shown the ground contact element 154s is made of rubber. The ground contact element 154d is implemented at least partly hollowly. The ground contact element 154d defines an interior space 318a. The tilt sensor 26d is at least partly, in particular entirely arranged within the interior space 218a.

The tilt sensor 26d comprises an actuation element 320d. The actuation element 320d is implemented as a lever. The actuation element 320d is swivel-mounted about a swivel axis 324d. The actuation element 320d is mounted to a foot unit support 148d.

The sensing unit 12d comprises a transmission element 322d. The transmission element 322d is connected to the actuation element 320d. The transmission element 322d is connected to a safety unit, which is not shown in fig. 13. The transmission element 322d is implemented as a pull cable.

The tilt sensor 26d comprises an activation element 326d. The activation element 326d is arranged within the ground contact element 154d. In an emergency condition the foot unit support 148d tilts backwards in a falling direction 328d. In the emergency condition the ground contact element 154d is partly deformed as a result of the tilting of the foot unit support 148d. In the emergency condition, a rear end portion 330d of the ground contact element 154d is deformed. In the emergency condition, the ground contact element 154d is pushed against the activation element 326d. In the emergency condition, the activation element 326d is pushed against the actuation element 320d. The actuation element 320d is swiveled about the swivel axis 324d in the emergency condition. The actuation element 320d exerts a pulling force onto the transmission element 322d in the emergency condition. The actuation element 320d is configured for partly transforming a pushing force exerted onto the activation element 326d into a pulling force exerted onto the transmission element 322d. The pulling force is configured for triggering a safety unit, in particular a deployment of a safety element of the safety unit.

It is conceivable that an actuation element is at least partly implemented integrally, or implemented integrally, with an activation element. Furthermore, other mechanical sensing units are conceivable for being used in a wearable sitting posture assisting device according to the invention. In particular, it is conceivable that an actuation element of a mechanical sensing unit is arranged outside a ground contact element. For instance, an actuation element may be implemented as push rod or the like which is in particular configured for directly contacting a ground in an emergency condition.

## Claims

1. Wearable sitting posture assisting device comprising at least one safety module (10a; 10; 10c), the safety module (10a; 10b; 10c) comprising at least one sensing unit (12a, 12b; 12c; 12d) configured for detecting at least one emergency condition, and the safety module (10a; 10b; 10c) comprising at least one safety unit (14a; 14b; 14c) configured for triggering at least one countermeasure for the emergency condition.

2. Wearable sitting posture assisting device according to claim 1, **characterized in that** the safety unit (14a; 14b; 14c) comprises at least one deployable safety element (16a; 16b; 16c) configured for at least partly carrying a weight of a person (200a) in the emergency condition.

3. Wearable sitting posture assisting device according to claim 2, **characterized in that** the safety element (16a; 16b; 16c) is at least partly self-extracting.

4. Wearable sitting posture assisting device according to claim 2 or 3, **characterized in that** the safety element (16a; 16b) comprises at least one extendable leg (18a; 18b).

5. Wearable sitting posture assisting device according to one of the preceding claims, **characterized in that** the safety element (16a; 16b; 16c) comprises at least one inflatable safety balloon (20a; 20b; 20c).

6. Wearable sitting posture assisting device at least according to claims 4 and 5, **characterized in that** the safety balloon (20a; 20b) is at least partly positioned within the extendable leg (18a; 18b) and is configured for generating at least one extension force for extending the extendable leg (18a; 18b).

7. Wearable sitting posture assisting device according to one of the preceding claims, **characterized in that** the safety element (16a; 16b) is swivel-mounted about at least one swivel axis (22a; 22b).

8. Wearable sitting posture assisting device according to claim 7, **characterized in that** the safety unit (14a; 14b) comprises at least one activation element (24a; 24b) configured for at least partly generating a swivel force on the safety element (16a; 16b) in the emergency condition.

9. Wearable sitting posture assisting device according to claim 8, **characterized in that** the activation element (24a) is pre-loadable by at least one movement applied to the safety element (16a).

10. Wearable sitting posture assisting device according to claim 8 or 9, **characterized in that** the activation element (24a) is implemented as a torsion spring.

11. Wearable sitting posture assisting device according to one of the preceding claims, **characterized in that** the sensing unit (12a; 12d) features at least one tilt detector (26a; 26d) and/or at least one acceleration detector (28a) for detecting the emergency condition.

12. Wearable sitting posture assisting device according to one of the preceding claims, **characterized in that** the sensing unit (12a) features at least one sitting detector (58a), which is configured for detecting at least one sitting condition, in which sitting condition a person (200a) is sitting on the wearable sitting posture assisting device.

13. Wearable sitting posture assisting device according to claim 12, **characterized by** at least one leg unit (102a; 102b; 102c), to which the safety unit (14a; 14b; 14c) is mounted.

14. Safety module (10a 10b; 10c) for a wearable sitting posture assisting device (100a; 100b; 100c) according to one of the preceding claims.

15. Method for providing safety for a person (200a), in particular in a fabrication line, a factory building, a service building or an office building, wherein the person (200a) is wearing a wearable sitting posture assisting device (100a), in particular a wearable sitting posture assisting device (100a) according to one of claims 1 to 13, **characterized in that** in at least one emergency condition at least one countermeasure for the emergency condition is triggered.
